(19) Europäisches Patentamt
European Patent Office
Office européen des brevets

(11) Veröffentlichungsnummer: **0 005 522**
**A1**

(12) # EUROPÄISCHE PATENTANMELDUNG

(21) Anmeldenummer: 79101452.5

(22) Anmeldetag: 11.05.79

(51) Int. Cl.²: C 07 J 3/00
A 61 K 31/56

(30) Priorität: 12.05.78 CH 5208/78

(43) Veröffentlichungstag der Anmeldung: 28.11.79 Patentblatt 79/24

(84) Benannte Vertragsstaaten: CH DE FR GB NL

(71) Anmelder: F.Hoffmann-La Roche & Co. Aktiengesellschaft
Abt. VIII-Pt
CH-4002 Basel(CH)

(72) Erfinder: Alig, Leo, Dr.
Liebrütistrasse 32
CH-4303 Kaiseraugst(CH)

(72) Erfinder: Fürst, Andor, Dr.
Magnolienpark 14
CH-4052 Basel(CH)

(72) Erfinder: Müller, Marcel, Dr.
Quellenweg 10
CH-4402 Frenkendorf(CH)

(72) Erfinder: Kerb, Ulrich, Dr.
Prinzregentenstrasse 7
D-1000 Berlin 31(DE)

(72) Erfinder: Wiechert, Rudolf, Prof. Dr.
Petzowerstrasse 5
D-1000 Berlin 39(DE)

(74) Vertreter: Lederer, Franz, Dr. et al,
Patentanwälte Dr. Lederer Franz Meyer-Roxlau Reiner F.
Lucile-Grahn-Strasse 22
D-8000 München 80(DE)

(54) **Neue Steroide, Verfahren zu deren Herstellung und diese Steroide enthaltende Arzneimittel.**

(57) Neue hormonal wirksame Steroide der Formel

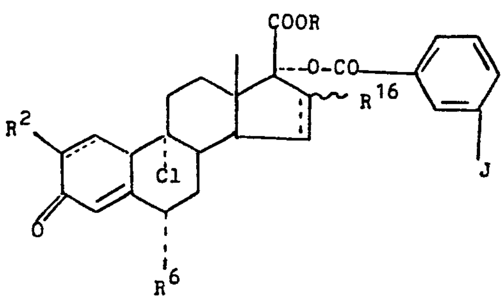

worin R Wasserstoff oder nieder-Alkyl, $R^2$ Wasserstoff oder Chlor, $R^6$ Wasserstoff, Fluor oder Methyl und $R^{16}$ Wasserstoff, α- oder β-Methyl, Methylen oder α-Hydroxy darstellen, die gestrichelten Bindungen im A- und D-Ring fakultativ sind, wobei jedoch der D-Ring nur wenn $R^{16}$ Methyl ist, ungesättigt sein kann,
und deren Herstellung ausgehend von den entsprechenden in 9-Stellung unsubstituierten 17α-Hydroxysteroiden.

EP 0 005 522 A1

0005522

11. Mai 1979

F. Hoffmann-La Roche & Co. Aktiengesellschaft, Basel, Schweiz

RAN 4104/160

# Neue Steroide, Verfahren zu deren Herstellung und diese Steroide enthaltende Arzneimittel

Die Erfindung betrifft neue Steroide der Formel

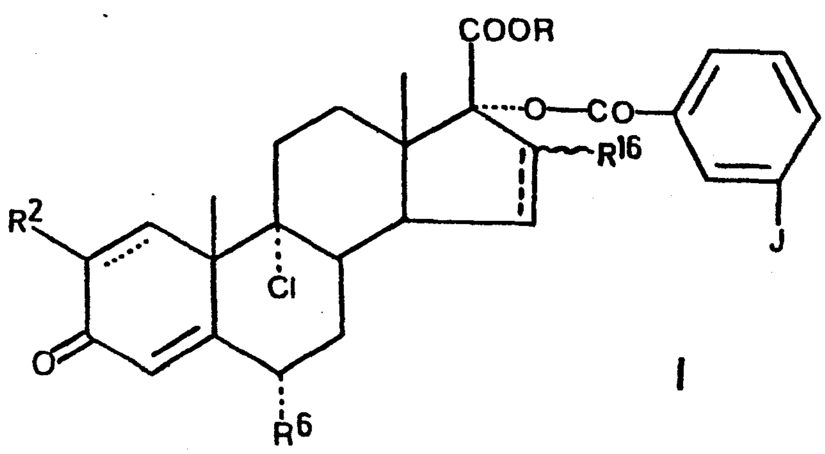

I

Mé/ 23.2.79

worin R Wasserstoff oder nieder-Alkyl, $R^2$ Wasserstoff oder Chlor, $R^6$ Wasserstoff, Fluor oder Methyl und $R^{16}$ Wasserstoff, α- oder β-Methyl, Methylen oder α-Hydroxy darstellen, die gestrichelten Bindungen im A- und D-Ring fakultativ sind, wobei jedoch der D-Ring nur wenn $R^{16}$ Methyl ist, ungesättigt sein kann.

Eine bevorzugte Gruppe von Steroiden der Formel I sind die Steroide, worin $R^2$, $R^6$ und $R^{16}$ Wasserstoff bedeuten.

Unter niederem Alkyl sind insbesondere solche Reste zu verstehen, die sich von niederen Alkanolen mit bis zu 4 Kohlenstoffatomen ableiten. Genannt seien beispielsweise Methyl, Aethyl, Propyl, Isopropyl, Butyl und Isobutyl.

Die erfindungsgemässen Verbindungen sind in erster Linie Zwischenprodukte zur Herstellung von pharmakologisch wertvollen Substanzen. Sie sind aber auch selbst schon pharmakologisch, z.B. hormonal, wirksam.

Beispiele von Verbindungen der Formel I sind

9α-Chlor-17-(m-jodbenzoyloxy)-3-oxo-androst-4-en-17β-carbonsäure,

2,9α-Dichlor-17-(m-jodbenzoyloxy)-3-oxo-androsta-1,4-dien-17β-carbonsäure,

9α-Chlor-6α-fluor-17-(m-jodbenzoyloxy)-3-oxo-androst-4-en-17β-carbonsäure,

9α-Chlor-6α-fluor-17-(m-jodbenzoyloxy)-3-oxo-androsta-1,4-dien-17β-carbonsäure,

9α-Chlor-17-(m-jodbenzoyloxy)-6α-methyl-3-oxo-androst-4-en-17β-carbonsäure,

9α-Chlor-17(m-jodbenzoyloxy)-6α-methyl-3-oxo-androsta-1,4-dien-17β-carbonsäure,

9α-Chlor-17-(m-jodbenzoyloxy)-16α-methyl-3-oxo-androst-4-en-17β-carbonsäure,

9α-Chlor-17-(m-jodbenzoyloxy)-16α-methyl-3-oxo-

androsta-1,4-dien-17β-carbonsäure,

9α-Chlor-17-(m-jodbenzoyloxy)-16β-methyl-3-oxo-androst-4-en-17β-carbonsäure,

9α-Chlor-17-(m-jodbenzoyloxy)-16β-methyl-3-oxo-androsta-1,4-dien-17β-carbonsäure,

9α-Chlor-17-(m-jodbenzoyloxy)-16-methyl-3-oxo-androst-4,15-dien-17β-carbonsäure,

9α-Chlor-17-(m-jodbenzoyloxy)-16-methyl-3-oxo-androsta-1,4,15-trien-17β-carbonsäure,

9α-Chlor-16α-hydroxy-17-(m-jodbenzoyloxy)-3-oxo-androst-4-en-17β-carbonsäure,

9α-Chlor-16α-hydroxy-17-(m-jodbenzoyloxy)-3-oxo-androsta-1,4-dien-17β-carbonsäure,

9α-Chlor-17-(m-jodbenzoyloxy)-16-methylen-3-oxo-androst-4-en-17β-carbonsäure,

9α-Chlor-17-(m-jodbenzoyloxy)-16-methylen-3-oxo-androsta-1,4-dien-17β-carbonsäure,

und die Methyl-, Aethyl-, Propyl- und Butylester dieser Verbindungen.

Nach Abspaltung des 9α-Chlors aus den Steroiden Formel I erhält man nach an sich bekannten Methoder z.B. mit Silberperchlorat in der Wärme, mit einem . oder Erdalkalimetallcarbonat in Dimethylformamid odeı organischen Base, wie Collidin, Lutidin und Pyridin, od einem Alkalihydroxyd in einem alkoholischen Lösungsmittel die entsprechenden $\Delta^{9(11)}$-Steroide. Bei der letzteren Methode werden gleichzeitig auch gegebenenfalls vorhandene Estergruppen gespalten. Die so erhaltenen $\Delta^{9(11)}$-Steroide sind wertvolle Ausgangsmaterialien für die Herstellung von bekannten 9α,11β-Dihalo-steroiden oder 11β-Hydroxy-steroiden, wie z.B. die aus den US Patentschriften 3828080 und 3856828 bekannten Steroide.

Die 9α,11β,Dihalosteroide werden bekanntlich so erhalten, dass man an die $\Delta^{9(11)}$-Verbindung Bromfluor, Chlorfluor oder Chlor addiert. Die 11β-Hydroxy-9α-fluor-steroide werden bekanntlich so erhalten, dass man an die entsprechende $\Delta^{9(11)}$-Verbindung unterbromige Säure addiert, das erhaltene 11β-Hydroxy-9α-brom-steroid durch Bromwasserstoffabspaltung in das 9β,11β-Oxido-steroid überführt und den Epoxidring mit Fluorwasserstoff wieder öffnet. Um zu den 11β-Hydroxy-steroiden zu gelangen, kann man auch das 11β-Hydroxy-9α-brom-steroid mit Tributylzinnhydrid, Raney-Nickel oder Chrom-II-chlorid debromieren.

Die erfindungsgemässen Verbindungen der Formel I haben insbesondere den Vorteil, dass sie einen leichten Zugang zu den 11β-Hydroxy-steroiden bieten, die bisher durch mikrobiologische Hydroxylierung hergestellt wurden. Während bei mikrobiologischen Methoden aufwendige Vorkehrungen getroffen werden müssen (Anzüchten der Mikroorganismen, Sterilität aller Arbeitsmittel, grosse Volumina), können die 11β-Hydroxy-steroide durch technisch einfach zu realisierende Verfahrensschritte ausgehend von den Steroiden der Formel I hergestellt werden.

Die Erfindung betrifft des weiteren ein Verfahren zur Herstellung von Verbindungen der Formel I, welches dadurch gekennzeichnet ist, dass man ein Steroid der Formel

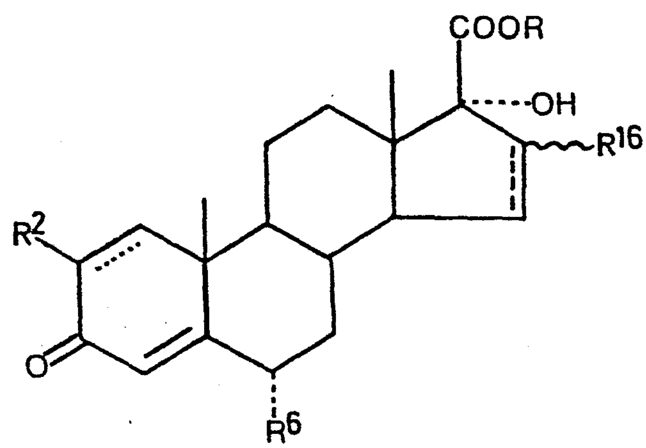

II

worin R, $R^2$, $R^6$, $R^{16}$ und die gestrichelten Bindungen im A- und D-Ring die gleiche Bedeutung wie in Formel I haben,

mit einem m-Jodbenzoylierungsmittel verestert, gewünschtenfalls eine freie 17β-Carboxygruppe verestert und den erhaltenen 17α-m-Jodbenzoylester mit Chlor, Sulfurylchlorid oder Jodbenzoldichlorid versetzt und mit langwelligem UV-Licht bestrahlt oder in Gegenwart eines Radikalbildners erhitzt und gewünschtenfalls eine freie 17β-Carboxygruppe verestert.

Aus den Arbeiten von Breslow et al., z.B. J. Amer. Chem. Soc. 96 (1974) 1973, ibid. 96 (1974) 6791 ist bekannt, dass man bei in 3α-Stellung veresterten Steroiden mit Jodbenzoldichlorid unter Lichteinfluss das tertiäre Kohlenstoffatom in 9-Stellung chlorieren und anschliessend Chlorwasserstoff unter Ausbildung einer 9,11-Doppelbindung wieder abspalten kann. Dieses Verfahren hat jedoch den Nachteil, dass es allein auf solche Steroide anwendbar ist, die keine Carboxygruppe oder keine ungeschützte Carbonylgruppe in der 17β-Seitenkette aufweisen. Nach dem erfindungsgemässen Verfahren werden jedoch selektiv 9α-Chlor-3-oxo-androst-4-en-17β-carbonsäuren erhalten. Es war ferner durchaus überraschend,dass die Chlorierung selektiv in 9-Stellung erfolgt, da aus den Arbeiten von Halpern, z.B. Chem. & Ind., 1962, 1571, bekannt war, dass Steroide mit Doppelbindungen mit Jodbenzoldichlorid zu den entsprechenden α-Dichlorsteroiden reagieren.

Das erfindungsgemässe Verfahren wird zweckmässigerweise so durchgeführt, dass man die Ausgangsstoffe der Formel II mit einem m-Jodbenzoylierungsmittel, wie m-Jodbenzoylchlorid oder -anhydrid, in Gegenwart eines säurebindenden Mittels, z.B. Pyridin oder Triäthylamin, oder in Gegenwart eines starken Säurekatalysators, z.B. p-Toluolsulfonsäure, umsetzt. Als Lösungsmittel für die m-Jodbenzoylierung kommen nicht-hydroxylgruppenhaltige organische Lösungsmittel, z.B. chlorierte Kohlenwasserstoffe, wie Methylen-

chlorid, oder Kohlenwasserstoffe, wie Benzol, in Betracht. Dabei entsteht zunächst ein gemischtes Anhydrid der Steroidcarbonsäure und der m-Jodbenzoesäure, welches sauer oder basisch, z.B. mit wässriger Essigsäure bzw. wässrigem Diäthylamin, zum gewünschten 17α-m-Jodbenzoyloxyderivat der Ausgangsverbindung II gespalten wird.

Die Ueberführung des so erhaltenen 17α-(m-Jodbenzoyl)-steroidesters in das entsprechende Steroid der Formel I wird zweckmässigerweise in einem geeigneten Lösungsmittel durchgeführt. Geeignete Lösungsmittel sind solche, die von dem verwendeten Halogenierungsmittel nicht angegriffen werden, wie halogenierte Kohlenwasserstoffe, z.B. Methylenchlorid, Chloroform, Tetrachlorkohlenstoff, Trichloräthylen oder Dichloräthylen, und aromatische Kohlenwasserstoffe, wie Benzol, Chlorbenzol oder Toluol, die gegebenenfalls auch als Mischungen untereinander verwendet werden können. Zweckmässigerweise wird die Reaktion unter Sauerstoffausschluss in einer Schutzgasatmosphäre ausgeführt. Hierzu leitet man ein inertes Gas, wie Stickstoff oder Argon, in die Reaktionslösung. Die Bestrahlung mit langwelligem UV-Licht kann mit einem im Handel erhältlichen Ultraviolettstrahler, z.B. einer Quecksilberhochdrucklampe, erzeugt werden. Als Radikalbildner können organische Peroxide, wie Dibenzoylperoxide, Cu-I-Acetat oder Azodiisobutyronitril, verwendet werden. Zweckmässigerweise werden 1 bis 25, vorzugsweise 10 Aequivalente des Radikalbildners für 100 Aequivalente des zu chlorierenden Steroids, in einem Lösungsmittel, z.B. einem der oben genannten halogenierten Kohlenwasserstoffe, verwendet.

Die Veresterung einer freien 17β-Carboxygruppe kann nach an sich bekannten Methoden, z.B. mit einem Diazoalkan, wie Diazomethan in Aether, oder durch Umsetzung eines Salzes der 17β-Carbonsäure, z.B. eines Alkalisalzes, mit einem Alkylhalogenid, wie Methyljodid, bewerkstelligt werden.

Die Ausgangsstoffe der Formel II können, soweit sie nicht bekannt oder nachstehend beschrieben sind, in Analogie zu bekannten oder in den Beispielen beschriebenen Methoden hergestellt werden.

Die Verbindungen der Formel I wirken hormonal, insbesondere auf das endokrine System und können demgemäss als hormonal wirksame Mittel, z.B. als Progestativa, verwendet werden und oral oder parenteral verabreicht werden. Als Dosierungsrichtlinie kommen 0,005 mg/kg bis 0,15 mg/kg pro die in Betracht.

Die Verbindungen der Formel I können als Heilmittel z.B. in Form pharmazeutischer Präparate Verwendung finden, welche sie in Mischung mit einem für die enterale, perkutane oder parenterale Applikation geeigneten organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle Stoffe enthalten.

## Beispiel 1

1 g 17-Hydroxy-3-oxo-androst-4-en-17β-carbonsäure wird in 30 ml Methylenchlorid in Gegenwart von 1,4 ml Triäthylamin mit 1 ml m-Jodbenzoylchlorid während 2 Stunden bei $0^{o}C$ umgesetzt. Das intermediäre gemischte Anhydrid wird durch Zugabe von wässeriger Natriumcarbonatlösung gespalten. Man erhält 1,48 g 17-(m-Jodbenzoyloxy)-3-oxo-androst-4-en-17β-carbonsäure, Smp. 235-236$^{o}C$, $\varepsilon_{220}$ = 33'600, $[\alpha]_D$ = $-22^{o}$ (Dioxan, c = 0.1%).

1.68 g 17-(m-Jodbenzoyloxy)-3-oxo-androst-4-en-17β-carbonsäure und 825 mg Jodbenzoldichlorid wurden in 340 ml Chloroform mit Argon begast und 20 Minuten mit einer Quecksilberhochdrucklampe belichtet. Die Lösung wurde mit verdünnter Natriumbisulfitlösung und Wasser gewaschen, getrocknet und im Vakuum eingedampft. Chromatographie an Kieselgel gab 9α-Chlor-17-(m-jodbenzoyloxy)-3-oxo-androst-4-en-17β-carbonsäure, Smp. 236-237$^{o}C$, $[\alpha]_D$ = $-40^{o}$ (c = 0.1% in Dioxan), $\varepsilon_{220}$ = 34'900.

1.02 g 9α-Chlor-17-(m-jodbenzoyloxy)-3-oxo-androst-4-en-17β-carbonsäure wurden in 100 ml Aceton mit 1 g Silberperchlorat in 30 ml Wasser versetzt und während 4 Std. unter Argon am Rückfluss gekocht. Nach Zugabe von 2 ml gesättigter Kochsalzlösung wurde das Reaktionsgemisch filtriert und im Vakuum eingeengt. Der Rückstand wurde mit Methylenchlorid verdünnt und mit verdünnter Kochsalzlösung gewaschen. Die getrocknete Lösung wurde eingedampft und die 17-(m-Jodbenzoyloxy)-3-oxo-androst—4,9(11)-dien-17β-carbonsäure aus Aceton kristallisiert, Smp. 248-249$^{o}C$, $[\alpha]_D$ = $-31^{o}$ (Dioxan, c = 0.1%), $\varepsilon_{220}$ = 36'000.

890 mg 17-(m-Jodbenzoyloxy)-3-oxo-androst—4,9(11)-dien-17β-carbonsäure wurden in 20 ml Aethylenglycol mit Argon begast, mit 4 g Kaliumhydroxyd versetzt und 2 1/2 Std. auf 110$^{o}C$ erwärmt. Nach Zugabe von 6 ml Essigsäure wurde das Reaktionsgemisch auf verdünnte Salzsäure gegossen und mit

Methylenchlorid extrahiert. Die Methylenchloridlösungen wurden mit Wasser und verdünnter Natriumchloridlösung gewaschen, getrocknet und im Vakuum eingedampft. Chromatographie an Kieselgel gab 17-Hydroxy-3-oxo-androst—4,9(11)-dien-17β-carbonsäure, Smp. 267-268°C, $\varepsilon_{240}$ = 17'240, $[\alpha]_D$ = +58° (Dioxan, c = 0.1%). Auf gleiche Weise erhält man auch aus 9α-Chlor-17-(m-jodbenzoyloxy)-3-oxo-androst-4-en-17β-carbonsäure direkt die 17-Hydroxy-3-oxo-androst—4,9(11)-dien-17β-carbonsäure.

## Beispiel 2

Aus 17-(m-Jodbenzoyloxy)-3-oxo-androst-4-en-17β-carbonsäure und Methyljodid erhält man in Dimethylacetamid in Gegenwart von Natriumhydrogencarbonat 17-(m-Jodbenzoyloxy)-3-oxo-androst-4-en-17β-carbonsäuremethylester, Smp. 190-191°C, $[\alpha]_D$ = -21° (Dioxan, c = 0.1%). $\varepsilon_{222}$ = 36'300.

In Analogie zu Beispiel 1 erhält man aus 17-(m-Jodbenzoyloxy)-3-oxo-androst-4-en-17β-carbonsäuremethylester den 9α-Chlor-17-(m-jodbenzoyloxy)-3-oxo-androst-4-en-17β-carbonsäuremethylester, Smp. 164-166°C, $[\alpha]_D$ = -31° (Dioxan, c = 0.1%), $\varepsilon_{221}$ = 33'200 und den 17-(m-Jodbenzoyloxy)-3-oxo-androst—4,9(11)-dien-17β-carbonsäuremethylester, Smp. 190-191°C, $[\alpha]_D$ -33° (Dioxan, c = 0.1%), $\varepsilon_{222}$ = 36'900.

410 mg 17-(m-Jodbenzoyloxy)-3-oxo-androst—4,9(11)-dien-17β-carbonsäuremethylester wurden in 15 ml 2-Methoxyäthanol mit Argon begast, mit 3 g Kaliumhydroxyd versetzt und 50 Minuten bei 80°C gerührt. Das Reaktionsgemisch wurde mit 4,5 ml Essigsäure neutralisiert, auf verdünnte Salzsäure gegossen und dreimal mit Methylenchlorid extrahiert. Die Methylenchloridlösungen wurden mit verdünnter Kochsalzlösung gewaschen, getrocknet und im Vakuum eingedampft. Chromatographie des Rückstandes an Kieselgel gab 17-Hydroxy-3-oxo-androst—4,9(11)-dien-17β-carbonsäure.

Diese kann unter den gleichen Reaktionsbedingungen auch aus

9α-Chlor-17-(m-jodbenzoyloxy)-3-oxo-androst-4-en-17β-carbonsäuremethylester erhalten werden.

Beispiel 3

In an sich bekannter Weise werden Tabletten folgender Zusammensetzung hergestellt:

| | |
|---|---|
| Wirkstoff, z.B. 9α-Chlor-17-(m-jodbenzoyloxy)-3-oxo-androst-4-en-17β-carbonsäuremethylester | 1 mg |
| Lactose | 60 mg |
| Stärke | 37 mg |
| Talkum | 1,8 mg |
| Magnesiumstearat | 0,2 mg |
| | 100,0 mg |

## Patentansprüche

1. D-Homosteroide der Formel

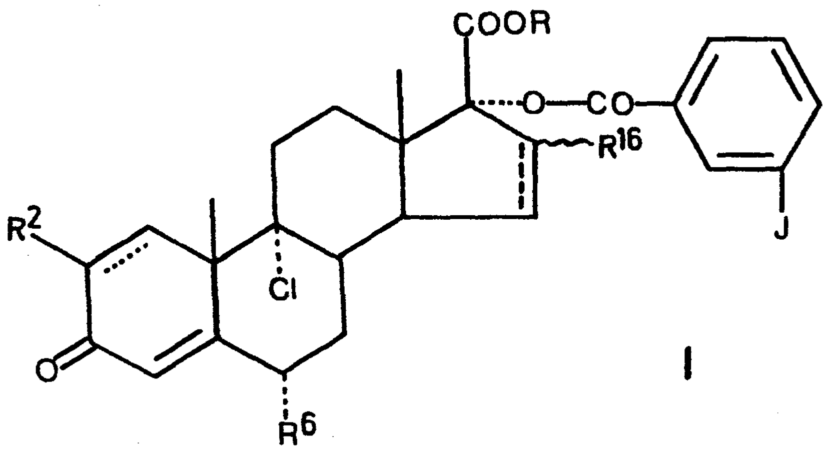

I

worin R Wasserstoff oder nieder-Alkyl, $R^2$ Wasserstoff oder Chlor, $R^6$ Wasserstoff, Fluor oder Methyl und $R^{16}$ Wasserstoff, α- oder β-Methyl, Methylen oder α-Hydroxy darstellen, die gestrichelten Bindungen im A- und D-Ring fakultativ sind, wobei jedoch der D-Ring nur wenn $R^{16}$ Methyl ist, ungesättigt sein kann.

2. 9α-Chlor-17-(m-jodbenzoyloxy)-3-oxo-androst-4-en-17β-carbonsäure und deren Methylester.

3. Verfahren zur Herstellung eines Steroids der Formel

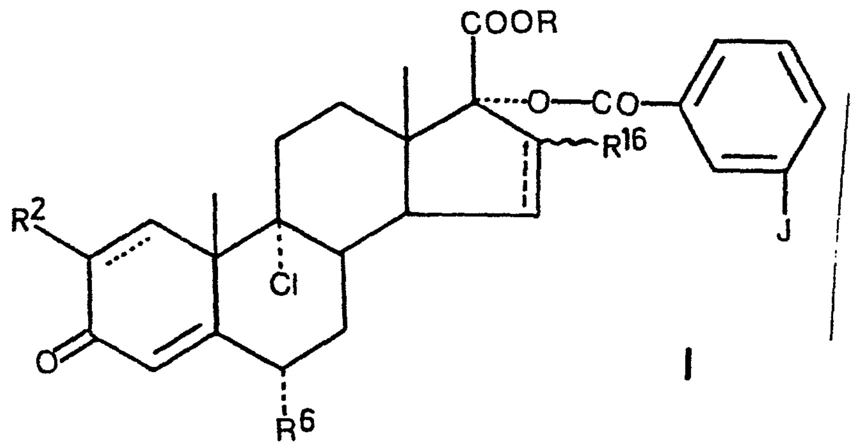

I

worin R Wasserstoff oder nieder-Alkyl, $R^2$ Wasserstoff oder Chlor, $R^6$ Wasserstoff, Fluor oder Methyl und $R^{16}$ Wasserstoff, α- oder β-Methyl, Methylen oder α-Hydroxy darstellen, die gestrichelten Bindungen im A- und D-Ring fakultativ sind, wobei jedoch der D-Ring nur wenn $R^{16}$ Methyl ist, ungesättigt sein kann,
dadurch gekennzeichnet, dass man ein Steroid der Formel

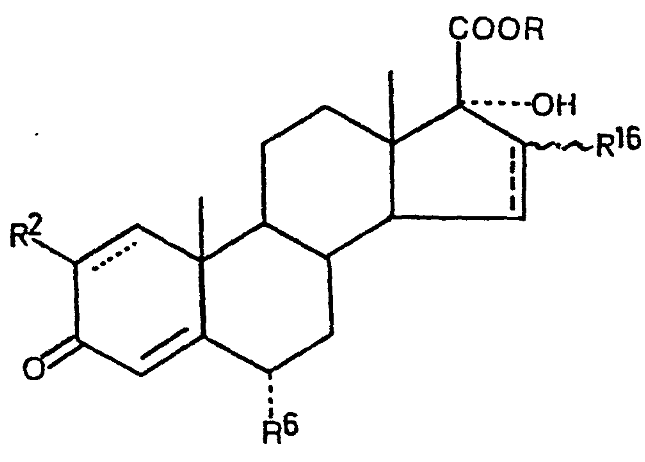

II

worin R, $R^2$, $R^6$, $R^{16}$ und die gestrichelten Bindungen im A- und D-Ring die gleiche Bedeutung wie in Formel I haben,

mit einem m-Jodbenzoylierungsmittel verestert, gewünschtenfalls eine freie 17β-Carboxygruppe verestert und den erhaltenen 17α-m-Jodbenzoylester mit Chlor, Sulfurylchlorid oder Jodbenzoldichlorid versetzt und mit langwelligem UV-Licht bestrahlt oder in Gegenwart eines Radikalbildners erhitzt und gewünschtenfalls eine freie 17β-Carboxygruppe verestert.

4. Arzneimittel, insbesondere hormonal aktives Mittel, erhaltend ein Steroid der Formel I.

***

0005522

Europäisches Patentamt

# EUROPÄISCHER RECHERCHENBERICHT

Nummer der Anmeldung

EP 79 10 1452

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| | JOURNAL OF THE AMERICAN CHEMICAL SOCIETY, vol. 99, nr. 3, 02-02-1977 Washington DC (USA) RONALD BRESLOW et al. "Selective halogenation of steroids using attached aryl iodide templates" Seiten 905-15. * Seite 910, verbindungen 23,24, Seite 913 und 914 * | 3 | C 07 J 3/00<br>A 61 K 31/56 |
| | -- | | |
| E | EP - A - 0 001 737 (SCHERING AG) * Anspruch 1 * | 3 | RECHERCHIERTE SACHGEBIETE (Int. Cl.³)<br>C 07 J 3/00<br>A 61 K 31/56 |
| | ---- | | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 24-07-1979 | HENRY |